# EUROPEAN PATENT APPLICATION

(11) **EP 4 591 855 A1**
(43) Date of publication of application: **30.07.2025**
(21) Application number: 24153534.3
(22) Date of filing: 23.01.2024
(51) Int. Cl.: A61K 9/10, A61K 31/00, A61K 47/08, A61K 47/14

(54) **STABLE DRONABINOL COMPOSITIONS**

(71) Applicant: Echo Pharmaceuticals B.V., 2333 CG Leiden (NL)
(72) Inventor: Iqbal, Muzamal, 2333 CG Leiden (NL); Kievid, Antony, 2333 CG Leiden (NL)
(74) Representative: Vossius & Partner Patentanwälte Rechtsanwälte mbB

(57) **Abstract**

The present invention relates to a composition comprising delta-9-tetrahydrocannabinol (THC, dronabinol), a lipophilic carrier such as medium chain triglycerides (MCT) and at least one additive. The present invention also relates to pharmaceutical compositions comprising said compositions. In particular, the present invention relates to a room temperature stable composition of THC in a lipophilic carrier. Furthermore, the present invention relates to a method for stabilizing THC in a composition and the use of specific additives for the stabilization of THC.

## Description

The present invention relates to a composition comprising delta-9-tetrahydrocannabinol (THC, dronabinol), a lipophilic carrier such as medium chain triglycerides (MCT) and at least one additive. The present invention also relates to pharmaceutical compositions comprising said compositions. In particular, the present invention relates to a room temperature stable composition of THC in a lipophilic carrier.

Furthermore, the present invention relates to a method for stabilizing THC in a composition and the use of specific additives for the stabilization of THC.

### BACKGROUND OF THE INVENTION

The cannabinoid delta- 9- tetrahydrocannabinol (also known as THC, dronabinol and Δ-⁹THC) 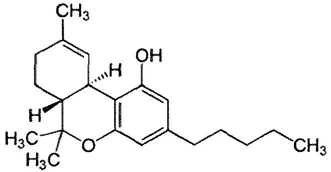 is naturally occurring substance and is the primary active ingredient of *Cannabis sativa.* It is a light-yellow resinous oil that is sticky at room temperature but hardens upon refrigeration. THC is mainly present in the flowers and leaves of the plant together with other minor cannabinoids that may help the patient with certain disease conditions. THC has been approved by the FDA i.a. for the control of nausea, vomiting associated with chemotherapy and for appetite stimulation for AIDS patients. It can also be chemically synthesized. THC is currently marketed under the trademarks Marinol^{®} (dronabinol capsules, USP), capsules containing THC solution in sesame oil and Syndros^{®}, an oral solution of THC 5 mg/ml in 50% (w/w) dehydrated alcohol and 5.5% (w/w) propylene glycol.

THC is known to deteriorate upon storage so that the effective concentration decreases upon storage and moreover, its purity is compromised due to the appearance of degradation products. Thus, preparations must be stored refrigerated, and if opened they can be stored only for a very limited amount of time, e.g. Syndros^{®}, where unused portions must be discarded 28 days after the first opening.

WO 2013/169406 A1 discloses an allegedly stable cannabinoid composition comprising an oral liquid solution of dronabinol comprising palmitoyl ascorbic acid in sesame oil which is encapsulated in capsules for increased stability.

The standardized formulations of Oily Dronabinol Solution NRF 22.8 and Dronabinol Capsules NRF 22.7 both comprise palmitoyl ascorbic acid as a stabilizer/antioxidant in MCT. Actually, MCT comprising palmitoyl ascorbic acid is a separate NRF monography (NRF S.45.).

Also, DE102012105063 A1 in particular discloses the stabilization of cannabinoids such as THC in oily carriers with palmitoyl ascorbic acid.

While corresponding compositions according to the NRF for direct use and as APIs for the formulation of magistral medicines are currently marketed i.a. by Cantourage GmbH in Germany, THC/Dronabinol for use in magistral medicines is usually packaged into 1ml glass syringes after purification steps and delivered to the pharmacies along with the products leaflets. Thereafter, it is heated at higher temperature until it becomes a flowable liquid and mixed with the oil and delivered to the patient. This process is not controlled and might lead to oxidation, degradation, and incorrect dosage as oxidizes dronabinol loses its therapeutic efficacy.

It should be understood that the content of any documents cited herein, while not necessarily considered relevant for the patentability of this invention, is incorporated by reference in its entirety. More specifically, all referenced documents are incorporated by reference to the same extent as if each individual document was specifically and individually indicated to be incorporated by reference.

To address the aforementioned problems, the present inventors have developed a composition and a method to provide the dronabinol in a stable formulation (which reduces/prevents oxidation and degradation of dronabinol.

Preferably, the present invention aims at avoiding the use of palmitoyl ascorbic acid as a stabilizer/antioxidant for oily compositions comprising THC, while still providing a stable composition, in particular a room temperature (RT) stable composition comprising THC. As palmitoyl ascorbic acid is very poorly soluble at room temperature, oily solutions need to be heated to 85°C to ensure complete solution and, thereby leading to instabilities. In accordance with the present invention, it is not necessary to heat the oily solutions of the stabilizers/antioxidants to 85°C to ensure complete solution, thereby instabilities and degradation may be avoided. Moreover, palmitoyl ascorbic acid is prone to precipitation if the compositions are stored at room temperature or below. Moreover, oily solutions comprising palmitoyl ascorbic acid, while initially odorless develop an unpleasant odor and taste over time.

### SUMMARY OF THE INVENTION

The present invention is directed to a composition comprising THC and at least one additive in a lipophilic carrier.

In one embodiment the invention provides a composition comprising THC from about 2.0 % (w/w) to about 50% (w/w) or from about 2.0 % (w/w) to about 25% w/w or, from about 2.0.% to about 20% (w/w) THC in a lipophilic carrier in a concentration of from about 46.0% (w/w) to about 97.99% (w/w), wherein the lipophilic carrier is selected from grape seed oil, peanut oil, rape seed oil (canola oil), safflower oil, sunflower oil, sesame oil, coconut oil, palm oil, palm kernel oil, caprylic acid/capric acid triglycerides and medium chain triglyceride (MCT) oil; and at least one an additive selected from the group consisting of (i) rosemary extract, (ii) a combination of carnosic acid and carnosol, (iii) clove extract, (iv) eugenol, (v) vitamin E(vi) a mixture of α-, β-, γ-, δ- tocopherol and (vii) lecithin in a concentration of about 0.01% w/w to about 2.0 % (w/w);
wherein % (w/w) refers to % w/w of the total composition and wherein the THC and the additives are dissolved in the lipophilic carrier.

In a preferred embodiment the lipophilic carrier is medium chain triglyceride (MCT) oil.

It is further preferred that the formulations as additives (stabilizers/antioxidants) comprise rosemary extract in an amount of about 0.01 to about 0.20 % (w/w) in combination with eugenol in an amount of about 0.01 to about 0.70% (w/w), or
rosemary extract in an amount of about 0.01% to about 0.20 % (w/w) in combination with vitamin E in an amount of about 0.01% to about 0.5 % (w/w), or lecithin in an amount of about 0.01% to about 1.0 % (w/w) in combination with vitamin E in an amount of about 0.01 to about 0.01% to 0.5% (w/w), whereby vitamin E is preferably alpha-tocopherol and more preferably synthetic vitamin E (DL-all-rac-α-tocopherol).

The use of a combination of rosemary extract in an amount of about 0.01% to about 0.20 % (w/w) in combination with vitamin E in an amount of about 0.01% to about 0.5 % (w/w) is even more preferred, whereby vitamin E is preferably alpha-tocopherol and more preferably synthetic vitamin E (DL-all-rac-α-tocopherol).

Further preferred embodiments of the present invention are also described in the appended claims.

### DETAILED DECRIPTION OF THE INVENTION

As already mentioned above, the present invention is directed to a stable composition comprising THC and at least one additive in a lipid carrier. In accordance with the present disclosure, the additive is sometimes also referred to as stabilizer or antioxidant.

Thus, the present invention in particular provides the following:
(1) A composition comprising:
(a) delta-9-tetrahydrocannabinol (THC) in a concentration of from about 2.0% to about 50% (w/w) or from about 2.0 % (w/w) to about 25% (w/w) or from about 2.0% to about 20% (w/w);
(b) a lipophilic carrier in a concentration of from about 46.0% (w/w) to about 97.99% (w/w), wherein the lipophilic carrier is selected from grape seed oil, peanut oil, rape seed oil (canola oil), safflower oil, sunflower oil, sesame oil, coconut oil, palm oil, palm kernel oil, caprylic acid/capric acid triglycerides and medium chain triglyceride (MCT) oil, and
(c) at least one additive selected from the group consisting of (i) rosemary extract, (ii) a combination of carnosic acid and carnosol, (iii) clove extract, (iv) eugenol, (v) vitamin E, (vi) a mixture of α-, β-, γ-, δ- tocopherol and (vii) lecithin in a concentration of about 0.01% (w/w) to about 2.0 % (w/w);
wherein % (w/w) refers to % (w/w) of the total composition and wherein the THC and the additives are dissolved in the lipophilic carrier.
(2) The composition according to (1), comprising
(a) delta-9-tetrahydrocannabinol (THC) in a concentration of from about 2.0 % (w/w) to about 25% (w/w), from about 2.5% to about 20% w/w, in particular about 2.1% (w/w) to about 20.0% (w/w), 2.2% (w/w) to about 15 % (w/w); about 2.3 % (w/w) to about 10 % (w/w), about 2.4 % (w/w) to about 7.5% (w/w) or about 2.5% (w/w) to about 5.0% w/w;
(b) a lipophilic carrier in a concentration of from about 73.0% (w/w) to about 97.99% (w/w);
(c) at least one an additive selected from the group consisting of (i) rosemary extract, (ii) a combination of carnosic acid and carnosol, (iii) clove extract, (iv) eugenol, (v) vitamin E, (vi) a mixture of α-, β-, γ-, δ- tocopherol and (viii) lecithin in a concentration of about 0.01% (w/w) to about 2.0 % (w/w).
(3) The composition according to (1) or (2), wherein the lipophilic carrier is medium chain triglyceride (MCT) oil.
(4) The composition according to any of (1) to (3) above, wherein the composition comprises about 2.0% (w/w) to about 10.0% (w/w) THC.
(5) The composition according to any of (1) to (4) above, wherein the composition comprises about 2.5 % w/w to about 5.0 % w/w THC.
(6) The composition according to any of (1) to (5) above, wherein the composition comprises about 2.5% (w/w) THC.
(7) The composition according to any of (1) to (6) above, wherein the composition comprises the additive in a concentration from about 0.01 % (w/w) to about 1.5 % (w/w).
(8) The composition according to any of (1) to (7) above, wherein the composition comprises one or more of rosemary extract at a concentration of 0.01% to 0.20% (w/w), eugenol at a concentration of about 0.01% to 0.70% (w/w), vitamin E at a concentration of 0.01% to 0.5% (w/w), a mixture of α-, β-, γ-, δ- tocopherol at a concentration of 0.01% to 0.5% (w/w) and/or lecithin at a concentration of 0.01% to 1.0% (w/w).
(9) The composition according to any of (1) to (8) above, wherein the additive comprises:
(i) rosemary extract and eugenol,
(ii) rosemary extract and vitamin E,
(iii) rosemary extract and a mixture of α-, β-, γ-, δ- tocopherol,
(iv) vitamin E, a mixture of α-, β-, γ-, δ- tocopherol and rosemary extract,
(v) lecithin, or
(vi) lecithin and vitamin E.
(10) The composition according to any of (1) to (9) above, wherein the additive consists of:
(i) rosemary extract and eugenol,
(ii) rosemary extract and vitamin E,
(iii) rosemary extract and a mixture of α-, β-, γ-, δ- tocopherol,
(iv) vitamin E, a mixture of α-, β-, γ-, δ- tocopherol and rosemary extract,
(v) lecithin, or
(vi) lecithin and vitamin E.
(11) The composition according to any of (1) to (9), wherein the additive comprises a first and a second component selected from
(i) rosemary extract as the first component and eugenol as the second component,
(ii) rosemary extract as the first component and vitamin E as the second component,
(iii) rosemary extract as the first component and a mixture of α-, β-, γ-, δ-tocopherol as the second component, or
(iv) lecithin as the first component and vitamin E as the second component.
(12) The composition according to any of (1) to (9) above, wherein the additive consists of a first and a second component selected from
(i) rosemary extract as the first component and eugenol as the second component,
(ii) rosemary extract as the first component and vitamin E as the second component,
(iii) rosemary extract as the first component and a mixture of α-, β-, γ-, δ-tocopherol as the second component, or
(iv) lecithin as the first component and vitamin E as the second component.
(13) The composition according to any of (1) to (12) above, wherein the additive is selected from:
(i) a combination of about 0.01% to 0.20% (w/w) rosemary extract and about 0.01% to 0.70% (w/w) eugenol,
(ii) a combination of about 0.01% to 0.20% (w/w) rosemary extract and 0.01% to 0.5% (w/w) vitamin E and
(iii) a combination of about 0.01% to 1.0% (w/w) lecithin and of about 0.01% to 0.5% (w/w) vitamin E.
(14) The composition according to any of (10) to (13) above, wherein the weight ratio of the first to second component of the additive is from about 3:2 to about 2:3, preferably about 1:1.
(15) The composition according to any one of the above, selected from a composition comprising
(a) about 2.5% (w/w) THC,
(b) MCT oil and
(c) a combination of rosemary extract and eugenol;
a composition comprising
(a) about 2.5 % (w/w) THC,
(b) MCT oil and
(c) a combination of rosemary extract and vitamin E; and
a composition comprising
(a) about 2.5% (w/w) THC,
(b) MCT oil and
(c) a combination of lecithin and vitamin E.
(16) The composition according to any of the above, wherein vitamin E is α-tocopherol.
(17) The composition according to any of the above, selected from
a composition comprising:

| | |
|---|---|
| THC | 2.5% (w/w), |
| Rosemary extract | 0.02% (w/w,) |
| Eugenol | 0.02% (w/w) and |
| MCT | 96.00 to 97.46% (w/w); |

a composition comprising:

| | |
|---|---|
| THC | 2.5% (w/w), |
| Rosemary extract | 0.02% (w/w), |
| Vitamin E (α-tocophero!) | 0.02% (w/w), and |
| MCT oil | 96.00 to 97.46% (w/w); |

a composition comprising:

| | |
|---|---|
| THC | 2.5% (w/w) |
| Rosemary extract | 0.05% (w/w) |
| Vitamin E (a-tocopherol) | 0.05% (w/w) and |
| MCT oil | 96.00 to 97.40% (w/w); |

a composition comprising:

| | |
|---|---|
| THC | 2.5% (w/w) |
| Lecithin | 0.02% (w/w) |
| Vitamin E (α-tocopherol) | 0.02% (w/w) and |
| MCT oil | 96.00 to 97.46% (w/w); and |

a composition comprising:

| | |
|---|---|
| THC | 2.5% (w/w) |
| Lecithin | 0.05% (w/w) |
| Vitamin E (α-tocopherol) | 0.05% (w/w) and |
| MCT oil | 96.00 to 97.40% (w/w). |

(18) The composition according to (17) above, selected from
a composition comprising:

| | |
|---|---|
| THC | 2.5% (w/w) |
| Rosemary extract | 0.05% (w/w) |
| Vitamin E (a-tocopherol) | 0.05% (w/w) |
| MCT oil | 96.00 to 97.40% (w/w), and |

a composition comprising:

| | |
|---|---|
| THC | 2.5% (w/w) |
| Lecithin | 0.02% (w/w) |
| Vitamin E (α-tocopherol) | 0.02% (w/w) and |
| MCT oil | 96.00 to 97.46% (w/w). |

(19) The composition according to any of the above, wherein vitamin E is synthetic vitamin E (DL-all-rac-α-tocopherol).
(20) The composition according to any of the above, wherein vitamin E is natural vitamin E (D-α-tocopherol).
(21) The composition according to any of the above, which does not comprise any further components.
(22) A composition according to any of the above for the use in medicine.
(23) A pharmaceutical composition comprising a composition according to any of the above and optionally further pharmaceutically active ingredients, preferably selected from other cannabinoids selected from cannabidiol (CBD), cannabinol (CBN), cannabigerol (CBG), cannabichromene (CBC) and Δ9-tetrahydrocannabutol (THC-C4), preferably formulated for oral administration.
(24) A pharmaceutical formulation according to (23) above formulated as an emulsion, soft capsule, hard capsule, oily solution or alcoholic solution.
(25) The non-medicinal use of a composition according to any of (1) to (21) above.
(26) A method of stabilizing THC in a composition comprising THC comprising:
(a) preparing a solution of one or more members of the group consisting of rosemary extract, a combination of carnosic acid and carnosol, clove extract, eugenol, vitamin E, a mixture of alpha, beta, gamma- and delta tocopherol and lecithin in a lipophilic carrier and
(b) purging the solution obtained in (a) with N₂;
(c) preparing a solution of THC with the solution obtained in (b) under heating to about 70°C±5°C and
(d) purging the solution obtained in (c) with N₂, wherein the concentration of the of one or more members of the group consisting of rosemary extract, a combination of carnosic acid and carnosol, clove extract, eugenol, vitamin E, a mixture of alpha, beta, gamma- and delta tocopherol and lecithin in the total composition is as defined in (c) according to (1) above.
(27) The use of one or more compound(s) as defined in (c) according to (1) above for the stabilization of THC.

### Definitions

The following definitions apply throughout the present specification, unless specifically indicated otherwise.

The term "about" as used herein preferably refers to ±10% of the indicated numerical value, more preferably to ±5% of the indicated numerical value, and in particular to the exact numerical value indicated. If the term "about" is used in connection with the endpoints of a range, it preferably refers to the range from the lower endpoint -10% of its indicated numerical value to the upper endpoint +10% of its indicated numerical value, more preferably to the range from of the lower endpoint -5% to the upper endpoint +5%, and even more preferably to the range defined by the exact numerical values of the lower endpoint and the upper endpoint. If the term "about" is used in connection with the endpoint of an open-ended range, it preferably refers to the corresponding range starting from the lower endpoint -10% or from the upper endpoint +10%, more preferably to the range starting from the lower endpoint -5% or from the upper endpoint +5%, and even more preferably to the open-ended range defined by the exact numerical value of the corresponding endpoint.

The term room temperature ("RT") as used in the context of the present invention denotes a temperature of 20°C±5°C, preferably 20°C.

As used in the present invention the terms "comprising", "which comprises" or equivalent expressions encompass three alternatives, namely (i) "comprising", (ii) "consisting essentially of" and (iii) "consisting of".

It is understood that, unless specified otherwise, "%" relates to "% (w/w)", i.e. percentages based on weight/weight. Furthermore, unless specified otherwise, specifications such as "% (w/w)" refer to the total weight of the total composition.

The term "additive" as used in the context of the present invention refers to a component which is selected from the group consisting of (i) rosemary extract, (ii) a combination of carnosic acid and carnosol, (iii) clove extract, (iv) eugenol, (v) vitamin E, (vi) a mixture of α-, β-, γ-, δ- tocopherol and (vii) lecithin.

In accordance with the present disclosure, the additive(s) is/are sometimes also referred to as stabilizer(s) or antioxidant(s).

As used herein, the terms "stabilizer", and "anti-oxidant", are recognized in the art and refer to synthetic or natural substances that prevent or delay the oxidative or free radical or photo-induced deterioration of a compound.

The term "stable" or "stability" as used herein with respect to dronabinol refers to a dronabinol composition that contains at least about 80% w/w, preferably at least about 90% w/w and more preferably at least about 95% w/w of the dronabinol in undegraded form after exposure of the compositions at 5 °C, for at least 6 to 12 months and/or; 25 °C /60% RH for at least 6 months; and/or 40 °C/75% RH for at least 3 to 6 months, or under in use conditions for at least 3 to 6 months.

### Dronabinol

The terms "dronabinol", "THC", or "Δ9 - THC" refer to "(-)-Δ 9-trans-tetrahydrocannabinol", which are used interchangeably, are meant to encompass naturally occurring dronabinol, synthetically derived dronabinol, and synthetically modified dronabinol. In particular "dronabinol" can be obtained from natural sources as i.a. described in EP2 844 243 A1 by Echo Pharmaceuticals.

The use of natural (-)-Δ 9-trans-tetrahydrocannabinol is preferred, more preferably dronabinol in conformity of the requirements of the DAC/NRF Monography (2021) for dronabinol.

### Vitamin E

Vitamin E as used in accordance with the present invention refers to α-tocopherol, in particular synthetic vitamin E (DL-all-rac-α-tocopherol) or natural vitamin E (D-α-tocopherol). α-tocopherol may also be used as α-tocopheryl acetate, i.e. as D-α tocopheryl acetate as the natural form and DL-α-tocopheryl acetate as the synthetic form. Thus, the term vitamin E also encompasses natural and synthetic α-tocopheryl acetate. The use of synthetic Vitamin E (DL-all-rac-α-tocopherol) or natural vitamin E (D-α-tocopherol) is preferred. The use of synthetic vitamin E (DL-all-ac-α-tocopherol) is particularly preferred.

### Mixture of tocopherols

The term "mixture of tocopherols" as used in accordance with the present invention refers to a mixture of naturally occurring tocopherols, in particular a mixture of alpha, beta, gamma-and delta tocopherol. In a preferred embodiment the mixture of tocopherols comprises > 70% mixed tocopherols, in particular <14% α- tocopherol, <7% β- tocopherol, 35 to 49 % γ-tocopherol and 7 to 21% δ- tocopherol. Such a mixed tocopherol concentrate is e.g. commercially available as Tocopherol 70, natural concentrated from All Organic Treasures GmbH, Wiggensbach. In the context of the present invention the use of this mixed tocopherol concentrate is particularly preferred.

### Rosemary extract

The term "rosemary extract" as used in the context of the present invention refers to an oily liquid extract from *Rosemary officinalis (Salvia rosmarinus).* Preferably, the term "rosemary extract" refers to an oily liquid extract obtained by CO₂ supercritical extraction from *Rosemary officinalis (Salvia rosmarinus)* comprising a mixture of several phenolic diterpenes such as carnosic acid, carnosol and rosmarinic acid, comprising more than 10% (w/w) of carnosic acid.

In further preferred embodiments the term "rosemary extract "according to the present invention refers to an a viscous oily liquid extract obtained by CO₂ supercritical extraction from *Rosemary officinalis (Salvia rosmarinus)* comprising more that 10% (w/w)), preferably about 14% (w/w), even more preferred more than 20% (w/w) carnosol acid (carnosic acid) and having an antioxidation power (AP) of more than 80,000 AU (Antioxidation Units by Gematria determined in accordance with DE 10 2005 026 133 B4).

In a most preferred embodiment, the rosemary extract comprises more than 10% carnosic acid, preferably about 14% (w/w), an essential oil content of below 4.0 %, a water content of below 1.0% (Karl Fischer) and an ethanol content of below 2.0 % (w/w) and exhibits an antioxidation power (AP) of more than 80,000 AU (Antioxidation Units by Gematria determined in accordance with DE 10 2005 026 133 B4).

Such an extract is e.g. commercially available as StoppOX RE 60, Organic- Rosemary Extract from All Organic Treasures GmbH, Wiggensbach.

Alternatively, a combination of purified carnosic acid (0.001 to 2.0% (w/w)) /carnosol (0.001 to 2.0% (w/w)) may be used. The use of a rosemary extract, however, is preferred.

### Eugenol

The term "eugenol" as used in the context of the present invention refers to natural or synthetic eugenol, preferably having a purity of ≥98%. Natural eugenol is preferred.

Alternatively, also a clove (*Syzygium aromaticum*) extract comprising 90 to 99.9% eugenol may be used, the amounts cited below for eugenol need to be corrected accordingly.

### Lecithin

The term lecithin as used in the context of the present invention refers to a mixture of glycerophospholipids including phosphatidylcholine, phosphatidylethanolamine, phosphatidylinositol, phosphatidylserine, and phosphatidic acid.

Lecithin may be i.a. selected from soy lecithin, egg lecithin, rapeseed lecithin, milk lecithin and sunflower lecithin, whereby soy lecithin, in particular non-GMO soy lecithin is preferred.

Lecithin can be used as powdered lecithin (de-oiled lecithin) such as available as L-α-Lecithin, Soybean, R&D grade from Sigma Adrich or liquid lecithin for ease of formulation liquid lecithin(s) may be used.

Liquid soy lecithin typically comprises phosphatidylcholine (PC) (12-16% w/w), phosphatidylethanolamine (PE) (8-13% w/w), inositol phosphatides (PI) (7-12% w/w), phosphatidic acid (PA) (3-6% w/w) and other phosphatides (2-3% w/w) as well as soybean oil (40% w/w).

Liquid soy lecithin is e.g. available as LEICO F 600 IPM.

### Medium chain triglycerides

The term "medium chain triglycerides" (MCT)" in the context of the present invention refers to a class of triglyceride oils that are derived from fatty acids that are preferably about 6 to about 12 carbons in length.

Thus, medium chain triglycerides (MCT) refer to triglycerides whose fatty acids have an aliphatic tail of 6- 12 carbon atoms. Fatty acids incorporated in MCT are called medium-chain fatty acids (MCFA). In triglycerides three fatty acid molecules are bound to a glycerol backbone. Per definition, in MCT at least two of these three fatty acids must be MCFAs.

"MCT oils" are oils containing predominantly said MCT. These terms refer to a respective mixture of different MCT that may contain a variety of MCFA. According to the invention any reasonable mixing ratio shall be covered by these terms.

Particularly preferred in accordance with the present invention is the use of MCT Oil Type V Ph. Eur. 11.0, (INCI: name Caprylic/Capric Triglyceride) an MCT-oil of vegetable origin, which is a mixture of triglycerides of saturated fatty acids, mainly of caprylic (octanoic) acid and capric (decanoic) acid. The fatty acids are obtained from the oil extracted from the hard, dried fraction of the endosperm of *Cocos nucifera L.* or from the dried endosperm *of Elaeis guineensis Jacq.* with a minimum content of 95.0 % of saturated fatty acids with 8 or 10 carbon atoms.

This MCT oil has a fatty acid composition comprising: 6:0 caproic acid % max. 2.0; 8:0 caprylic acid % 50.0 - 80.0; 10:0 capric acid % 20.0 - 50.0; 12:0 lauric acid % max. 3.0; 14:0 myristic acid % max. 1.0; > = C16 % max. 1.0 (Ph. Eur. [2.4.22].

MCT oil is also e.g. commercially available as Miglyol 812, Miglyol 810, Captex 355 and Neobee M-5.

### Embodiments

It is to be understood that the present invention specifically relates to each, and every combination of features and embodiments described herein, including any combination of general and/or preferred features/embodiments.

The present invention relates to a composition comprising Dronabinol/ (-)-Δ 9-trans-tetrahydrocannabinol (THC) in concentrations ranging from about 2.0 % (w/w) to about 50% (w/w) or between about 2.0% (w/w) and about 25 % (w/w) or between about 2.0% to about 20% (w/w), and (a) stabilizing additive(s)/antioxidant(s) in concentrations ranging from about 0.01% (w/w) to about 2.0 % (w/w) or between 0.02% (w/w) and 1.0% (w/w) in an oily carrier such as a mixture of medium chain triglycerides (MCT).

Preferably, the oily carrier is MCT oil.

The amount of (-)-Δ 9-trans-tetrahydrocannabinol in the composition is from about 2.0% to about 50%, preferably from about 2.0% to about 25.0% as a weight percentage of the composition, or from about 2.0 % to about 20.0%, 2.5% to 20.0 %, about 2.5% to about 15.0%, about 2.5% to about 10%, about 2.5% to about 7.5%, about 2.5% to about 5.0% (w/w).

In preferred embodiments, the amount of (-)-Δ 9-trans-tetrahydrocannabinol in the composition is about 2.0,2.1, 2.2, 2.3, 2.4, 2.5, 2.6, 2.7, 2.8, 2.9, 3.0, 3.1, 3.2, 3.3,3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10,4, 10.5, 10.6, 10.7, 10.8, 10.9, 11.0, 11,1, 11.2, 11.3, 11.4, 11.5, 11.6, 11.7, 11.8, 11.9, 12.0, 12.1, 12.2, 12.3, 12.4, 12.5, 12. 6, 12.7, 12.8, 12.9, 13.0, 13.1, 13.2, 13.3, 13.4, 13.5, 13.6, 13.7, 13.8, 13.9, 14.0, 14.1, 14.2, 14.3, 14.4, 14.5, 14.6, 14.7, 14.8, 14.9, 15.0, 15.1, 15.2, 15.3, 15.4, 15.5, 15.6, 15.7, 15.8, 15.9, 16.0, 16.1, 16.2, 16.3, 16..4, 16.5, 16.6, 16.7, 16.8, 16.9, 17.0, 17.1, 17.2, 17.3, 17.4, 17.5, 17.6, 17.7,, 17.8, 17.9, 18.0, 18.1, 18.2, 18.3, 18.4, 18.5, 18.6, 18.7, 18.8, 18.9, 19.0, 19.1, 19.2, 19.3, 19.4, 19.4, 19.5, 19.6, 19.7, 19.8, 19.9, 20.1, 20.2, 20.3, 20.4, 20.5, 20.6, 20.7, 20.8, 20.9, 21.0, 21.1, 21.2, 21.2, 21.3, 21.4, 21.5, 21.6, 21.7, 21.8, 21.9, 22.0, 22.1, 22.2, 22.3, 22.4, 22.5, 22.6, 22.7, 22.8, 22.9, 23.0, 23.1, 23.2, 23.3, 23.4, 23.5, 23.6, 23.7, 23.7, 23.8, 23.9, 24.0, 24.1, 24.2, 24.3, 24.4, 24.5, 24.6, 26.7, 24.8, 24.9, 25.0, 25.1, 25.2, 25.2, 25.3, 26.3, 26.4, 26.5, 26.6, 26.7, 26.8, 26.9, 27.0, 27.1, 27.2, 27.3, 27.4, 27.5, 27.6, 27.7, 27.8, 27.9,28.0, 28.1, 28.2, 28.3, 28.3, 28.4, 28.5, 28.6, 28.7, 28.8, 28.9, 30.0, 30.1, 30.2, 30.3, 30.4, 30.5, 30.6, 30.7, 30.8, 30.9,31.0, 31.1, 31.2, 31.3, 31.4, 31.5, 31.6, 31.7, 31.8, 31.9, 32.0, 32.1, 32.2, 32.3, 32.4, 32.5, 32.6, 32.7, 32.8, 32.9, 33.0, 33.1, 33.2, 33.3, 33.4, 33.5, 33.6, 33.7, 33.8, 33.9, 34.0, 34.1, 34.2, 34.3, 34.4, 34.5, 34.6, 34.7, 34.8, 34.9, 35.0, 35.1, 35.2, 35.3, 35.4, 35.5, 35.6, 35.7, 35.8, 35.9, 36.0, 36.1, 36.2, 36.3, 36.4, 36.5, 36.6, 36.7, 36.8, 36.9, 37.0, 37.1, 37.2, 37.3, 37.4, 37.5, 37.6, 37.7, 37.8, 37.9, 38.0, 38.1, 38.2, 38.3, 38.4, 38.5, 38.6, 38.7, 38.8, 38.9, 39.0, 39.1, 39.2, 39.3, 39.4, 39.5, 39.6, 39.7, 39.8, 39.9, 40.0, 40.1, 40.2, 40.3, 40.4, 40.5, 40.6, 40.7, 40.8, 40.9, 41.0, 41.1, 41.2, 41.3, 41.4, 41.5, 41.5, 41.6, 41.7, 42.8, 42.9, 43.0, 43.1, 43.2, 43.3, 43.4, 43.5, 43.6, 43.7, 43.8, 43.9, 44.0, 44.1, 44.2, 44.3, 44.4, 44.5, 44.6, 44.7, 44.8, 44.9, 45.0, 45.1, 45.2, 45.3, 45.4, 45.5, 45.6, 45.7, 45.8, 46.9, 46.0, 46.1, 46.2, 46.3, 46.4, 46.5, 46.6, 46.7, 46.8, 46.9, 47.0, 47.1, 47.2, 47.3, 47.4, 47.5, 47.6, 47.7, 47.8, 47.9, 48.0, 48.1, 48.2, 48.3, 48.4, 48.5, 48.6, 48.7, 47.8, 48.9, 49.0, 49.1, 49.2, 49.3, 49.4, 49.5, 49.6, 49.7, 49.8, 49.9 or 50% (w/w).

In a more preferred embodiment, the amount of (-)-Δ 9-trans-tetrahydrocannabinol in the composition is from about 2.5% to about 5.0% (w/w), in an even more preferred embodiment the amount of (-)-Δ 9-trans-tetrahydrocannabinol is about 2.5% as a weight percentage of the composition and in the most preferred embodiment the amount of (-)-Δ 9-trans-tetrahydrocannabinolis 2.5% as a weight percentage of the total composition.

In additional preferred embodiments the amount of (-)-Δ 9-trans-tetrahydrocannabinol in the composition is about 20% (w/w), 40% (w/w) or 50% (w/w) of the composition.

The amount of rosemary extract in the composition is preferably from about 0.005 to about 0.25%, about 0.01 to about 0.20%, about 0.015 to about 0.1%, about 0.02% to about 0.05% (w/w).

In preferred embodiments, the amount of rosemary extract in the composition is about 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19 or 0.20 % (w/w) of the total composition. Particularly preferred are 0.02, 0.03, 0.04 and 0.05 % (w/w), even more preferred are 0.02 and 0.05% (w/w), with 0.05% (w/w) being most preferred.

Also corresponding amounts of mixtures of purified carnosic acid (0.001 % to 2.0% (w/w)) and carnosol (0.001% to 2.0% w/w) may be used.

However, the use of rosemary extract is preferred.

The amount of eugenol or clove extract (comprising 90-99% eugenol) is preferably from about 0.005 to about 0.75%, about 0.01 to about 0.70%, about 0.15 to about 0.65% (w/w).

In preferred embodiments, the amount of eugenol or clove extract is about 0.01, 0.02, 0.03, 0.04, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, 0.25, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.50, 0.51, 0.52, 0.53, 0.54, 0.55, 0.56, 0.57, 0.58, 0.59, 0.60, 0.1, 0.62, 0.63, 0.64, 0.65, 0, 66, 0.67, 0.68, 0.69 or 0.70% (w/w).

The use of eugenol is preferred. The use of 0.02% eugenol (w/w) is most preferred.

The amount of vitamin E (D-α-tocopherol or DL-all-rac-α-tocopherol) is preferably about 0.005 to about 0.55%, about 0.01% to about 0.50%, about 0.015% to about 0.2%, about 0.02% to about 0. 05% of the total composition.

In preferred embodiments, the amount of vitamin E is about 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49 or 0.50 % (w/w) of the total composition.

The amount of vitamin E acetate is preferably about 0.01 to about 0.55%, about 0.02% to about 0.50%, about 0.03% to about 0.4%, about 0.04% to about 0.05% of the total composition.

In preferred embodiments, amount of vitamin E acetate is about 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, or 0.50 % (w/w) of the composition.

The use of DL-all-rac-α- tocopherol (synthetic vitamin E) is preferred, the use of 0.02% to 0.05%, 0.02%, 0.03%, 0.04% or 0.05% DL-all-rac--α- tocopherol is more preferred, the use of 0.02% or 0.05% (w/w) DL-all-rac-α- tocopherol is even more preferred, with 0.05% (w/w) being most preferred.

The amount of the mixture of α-, β-, γ-, δ- tocopherol is preferably about 0.005% (w/w) to about 0.55% (w/w), about 0.01% (w/w) to about 0.50% (w/w), about 0.015% (w/w) to about 0.2% (w/w), about 0.02% (w/w) to about 0.05% (w/w).

In preferred embodiments, the amount of the mixture of α-, β-, γ-, δ-tocopherol is about 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, 0.25, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49 or 0.50 % (w/w) of the composition.

The use of 0.02% to 0.05 %, 0.02%, 0.03%, 0.04% or 0.05% of a mixture of α-, β-, γ-, 6-tocopherol is most preferred.

The amount of lecithin is preferably from about 0.005 to about 1.10%, about 0.01 to about 1.00%, about 0.02 to about 0.04% (w/w) of the total composition.

In preferred embodiments, the amount of lecithin about 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, 0.25, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.50, 0.51, 0.52, 0.52, 0.53, 0.54, 0.55, 0.56, 0.57, 0.57,, 0.58, 0.59, 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, 0.99 or 1.00 % of the formulation. The use of 0.02% to 0.04% (w/w) is more preferred. The use of 0.02%, 0.04% or 0.05% (w/w) is most preferred.

The above amounts cited for lecithin and as well as cited in the appended claims refer to powdered (de-oiled) pure lecithin, if liquid lecithin such as fluid soy lecithin LECICO F 6000 IPM is used the amounts need to be correspondingly adjusted.

If a mixture of two antioxidant components is used, the ratio of the two components is about 3:2 to about 2:3, preferably about 1:1.

In accordance with the present invention the following combinations of additives as antioxidants for stabilizing the composition comprising THC is preferred:
- a combination of rosemary extract (comprising more than 10% carnosic acid), in particular at a concentration of 0.01% to 0.20% (w/w) and eugenol, in particular at a concentration of 0.01% to 0.70% (w/w);
- a combination of rosemary extract, particular at a concentration of 0.01% to 0.20% (w/w) and vitamin E, in particular at a concentration of 0.01% to 0.5% (w/w);
   and
- a combination of vitamin E, in particular at a concentration of 0.01% to 0.5% (w/w), and lecithin, in particular at a concentration of 0.01% to 1.0% (w/w);
whereby vitamin E is preferably alpha-tocopherol and more preferably synthetic vitamin E (DL-all-rac-α-tocopherol).

The following combinations of additives as antioxidants for stabilizing the composition comprising THC is more preferred:
- a combination of rosemary extract, at a concentration of about 0.015 to about 0.1% (w/w) and vitamin E, at a concentration of about 0.015% to about 0.15% (w/w);
   and
- a combination of vitamin E, at a concentration of about 0.015% to about 0.15% (w/w), and lecithin, at a concentration of about 0.015% to about 0.5% (w/w);
whereby vitamin E is preferably alpha-tocopherol and more preferably synthetic vitamin E (DL-all-rac-α-tocopherol).

The following combinations of additives as antioxidants for stabilizing the composition comprising THC is even more preferred:
- a combination of rosemary extract (0.02% (w/w)) and eugenol (0.02% (w/w)),
- a combination of rosemary extract (0.05% (w/w)) and vitamin E (0.05% (w/w)),
- a combination of rosemary extract (0.02% (w/w)) and vitamin E (0.02% (w/w)),
- a combination of lecithin (0.02% (w/w)) and vitamin E (0.02% (w/w)); or
- a combination of lecithin (0.05% (w/w)) and vitamin E (0.05% (w/w));
whereby vitamin E is preferably alpha-tocopherol and more preferably natural vitamin E (D-α-tocopherol).

The following combinations of additives as antioxidants for stabilizing the composition comprising THC is most preferred:
- a combination of rosemary extract (0.05% (w/w)) and vitamin E (0.05% (w/w)) and
- a combination of rosemary extract (0.02% (w/w)) and vitamin E (0.02% (w/w)), whereby vitamin E is preferably alpha-tocopherol and more preferably synthetic vitamin E (DL-all-rac-α-tocopherol).

Preferred embodiments according to the present invention are as follows:
(a) A composition comprising
   - 2.5% -20% dronabinol/THC,
   - a lipophilic carrier,
      wherein the lipophilic carrier is MCT oil
   - 0.01% to 0.20% rosemary extract (containing more than 10% w/w carnosic acid) and
   - 0.01% to 0.70% eugenol,
   wherein the dronabinol, rosemary extract and eugenol are dissolved in the lipophilic carrier (MCT oil) and % relates to % (w/w) of the total composition.
(b) A composition comprising
   - 2.5% -20% dronabinol/THC,
   - a lipophilic carrier,
      wherein the lipophilic carrier is MCT oil
   - 0.01% to 0.20% rosemary extract (containing more than 10% w/w carnosoic acid) and
   - 0.01% to 0.5% vitamin E,
   wherein the dronabinol, rosemary extract and vitamin E are dissolved in the lipophilic carrier (MCT oil) and % relates to % (w/w) of the total composition, whereby vitamin E is preferably alpha-tocopherol and more preferably synthetic vitamin E (DL-all-rac-α-tocopherol).
(c) A composition comprising
   - 2.5% -20% dronabinol/THC,
   - a lipophilic carrier,
      wherein the lipophilic carrier is MCT oil,
   - 0.01% to 1.0% lecithin and
   - 0.01% to 0.5% vitamin E,
   where the dronabinol, lecithin and vitamin E are dissolved in the lipophilic carrier (MCT oil) and % relates to % (w/w) of the total composition, whereby vitamin E is preferably alpha-tocopherol and more preferably synthetic vitamin E (DL-all-rac-α-tocopherol).

Whereby a composition comprising
- 2.5% -20% dronabinol/THC,
- a lipophilic carrier,
   wherein the lipophilic carrier is MCT oil,
- 0.01% to 0.20% rosemary extract (containing more than 10% w/w carnosoic acid) and
- 0.01% to 0.5% vitamin E,
wherein the dronabinol, rosemary extract and vitamin E are dissolved in the lipophilic carrier (MCT oil) and % relates to % (w/w) of the total composition is more preferred, whereby vitamin E is preferably alpha-tocopherol and more preferably synthetic vitamin E (DL-all-rac-α-tocopherol).

Even more preferred embodiments of the present invention are the following:
(d) A composition comprising
   - 2.5% dronabinol/THC
   - a lipophilic carrier,
      wherein the lipophilic carrier is MCT oil,
   - 0.02% rosemary extract (containing more than 10% (w/w) carnosic acid) and
   - 0.02% eugenol,
   wherein the dronabinol, rosemary extract and eugenol are dissolved in the lipophilic carrier (MCT oil) and % relates to % (w/w) of the total composition, whereby vitamin E is preferably alpha-tocopherol and more preferably synthetic vitamin E (DL-all-rac-α-tocopherol).
(e) A composition comprising
   - 2.5% dronabinol/THC,
   - lipophilic carrier,
      wherein the lipophilic carrier is MCT oil,
   - 0.02% rosemary extract (containing more than 10 % w/w carnosic acid) and
   - 0.02% vitamin E,
   wherein the dronabinol, rosemary extract and vitamin E are dissolved in the lipophilic carrier (MCT oil) and % relates to % (w/w) of the total composition, whereby vitamin E is preferably alpha-tocopherol and more preferably synthetic vitamin E (DL-all-rac-α-tocopherol).
(f) A composition comprising
   - 2.5% dronabinol/THC,
   - lipophilic carrier,
      wherein the lipophilic carrier is MCT oil,
   - 0.05% rosemary extract (containing more than 10 % w/w carnosic acid) and
   - 0.05% vitamin E,
   wherein the dronabinol, rosemary extract and vitamin E are dissolved in the lipophilic carrier (MCT oil) and % relates to % (w/w) of the total composition, whereby vitamin E is preferably alpha-tocopherol and more preferably synthetic vitamin E (DL-all-rac-α-tocopherol).
(g) A composition comprising
   - 2.5% dronabinol/THC,
   - a lipophilic carrier,
      wherein the lipophilic carrier is MCT oil,
   - 0.02% lecithin and
   - 0.02% vitamin E,
   where the dronabinol, lecithin and vitamin E are dissolved in the lipophilic carrier (MCT oil) and % relates to % (w/w) of the total composition, whereby vitamin E is preferably alpha-tocopherol and more preferably synthetic vitamin E (DL-all-rac-α-tocopherol).
(h) A composition comprising
   - 2.5% dronabinol/THC,
   - a lipophilic carrier,
      wherein the lipophilic carrier is MCT oil,
   - 0.05% lecithin and
   - 0.05% vitamin E,
   where the dronabinol, lecithin and vitamin E are dissolved in the lipophilic carrier (MCT oil) and % relates to % (w/w) of the total composition, whereby vitamin E is preferably alpha-tocopherol and more preferably synthetic vitamin E (DL-all-rac-α-tocopherol).

Wherein a composition comprising
- 2.5% dronabinol/THC,
- lipophilic carrier,
   wherein the lipophilic carrier is MCT oil,
- 0.02% rosemary extract (containing more than 10 % w/w carnosic acid) and
- 0.02% vitamin E,
wherein the dronabinol, rosemary extract and vitamin E are dissolved in the lipophilic carrier (MCT oil) and % relates to % (w/w) of the total composition, whereby vitamin E is preferably alpha-tocopherol and more preferably synthetic vitamin E (DL-all-rac-α-tocopherol). or
a composition comprising
- 2.5% dronabinol/THC,
- lipophilic carrier,
   wherein the lipophilic carrier is MCT oil,
- 0.05% rosemary extract (containing more than 10 % w/w carnosic acid) and
- 0.05% vitamin E,
wherein the dronabinol, rosemary extract and vitamin E are dissolved in the lipophilic carrier (MCT oil) and % relates to % (w/w) of the total composition, whereby vitamin E is preferably alpha-tocopherol and more preferably synthetic vitamin E (DL-all-rac-α-tocopherol),
is most preferred.

In accordance with the present invention the use of synthetic vitamin E is preferred.

The compositions according to the present invention may be used in medicine, thus, the present invention also provides pharmaceutical compositions, which may either be the compositions described above as such or comprise the compositions described above.

The pharmaceutical compositions according to the present invention are preferably formulated as dosage forms for oral administration. Thus, the compositions according to the present invention may be included in common cosmetic and pharmaceutical formulations known to the person of ordinary skills in the art (see, e.g., Bauer et al., Pharmazeutische Technologie, 5. Edt. Govi-Verlag Frankfurt, 1997; Rudolf Voigt, Pharmazeutische Technologie, 9. Edt., Deutscher Apotheker Verlag Stuttgart, 2000 or Remington's Pharmaceutical Sciences, 17th ed., 1985, Mack Publishing Co.), such as oily solutions, alcoholic solution, emulsions (O/W, W/O, W/O/W or O/W/O) or incorporated in hard or soft capsules, e.g. in tamper proof capsules for oral use.

However, the compositions according to the present invention may also formulation e.g. for topical use as O/W and W/O creams, O/W and W/O emulsions (milk), ointments, tonic (lotion), gels, multiple emulsions (W/O/W and O/W/O) or simple solutions (oily or aqueous) (see, e.g., Bauer et al., Pharmazeutische Technologie, 5. Edt. Govi-Verlag Frankfurt, 1997; Rudolf Voigt, Pharmazeutische Technologie, 9. Edt., Deutscher Apotheker Verlag Stuttgart, 2000 and

Remington's Pharmaceutical Sciences, 17th ed., 1985, Mack Publishing Co.).

The pharmaceutical compositions according to the present invention are preferably formulated as dosage forms for oral administration. As such the pharmaceutical composition provided by the present invention may e.g. formulated as an oily solution, an alcoholic solution, an emulsion (O/W, W/O, W/O/W or O/W/O) or incorporated in hard or soft capsules, e.g. in tamper proof capsules.

The pharmaceutical and cosmetic compositions according to the present invention, in addition to the usual excipients well known to a person of skill in the art, may comprise additional ingredients such as other cannabinoids selected from cannabidiol (CBD), cannabinol (CBN), cannabigerol (CBG), cannabichromene (CBC) and Δ9-tetrahydrocannabutol (THC-C4) as loose combinations, fixed combinations or as part of a kit.

The compositions according to the present invention and pharmaceutical compositions comprising the same may be used in the prophylaxis and/or treatment of disorders or diseases such as chronic or neuropathic pain, painful muscle spasms in multiple sclerosis and paraplegia, epilepsy, Tourette's syndrome, depression, anxiety disorders and sleep disorders as well as in the management and treatment of chemotherapy-induced nausea and vomiting (CINV) and for the stimulation of appetite in patients with AIDS-related anorexia).

Furthermore, the present invention also provides a method of stabilizing dronabinol/THC in compositions comprising dronabinol/THC, wherein one or more members of the group consisting of rosemary extract, a combination of carnosic acid and carnosol, clove extract, eugenol, vitamin E (natural or synthetic), a mixture of alpha, beta, gamma- and delta tocopherol and lecithin are added to dronabinol/THC or compositions comprising dronabinol/THC, in particular to an oily solution of dronabinol/THC in MCT oil.

Thus, the present invention preferably provides a method for stabilizing THC in a lipophilic carrier, characterized in that one or more additives, members of the group consisting of rosemary extract, a combination of carnosic acid and carnosol, clove extract, eugenol, vitamin E (natural or synthetic), a mixture of alpha, beta, gamma- and delta tocopherol and lecithin; and the carrier consist of a lipophilic carrier selected from grape seed oil, peanut oil, rape seed oil (canola oil), safflower oil, sunflower oil, sesame oil, coconut oil, palm oil, palm kernel oil, caprylic acid/capric acid triglycerides and medium chain triglyceride (MCT) oil, and THC and the one or more additives are dissolved in the lipophilic carrier.

In a particularly preferred method according to the present invention, the lipophilic carrier is MCT oil, and the additives are selected from
(i) about 0.01% to 0.20% (w/w) rosemary extract and about 0.01% to 0.70% (w/w) eugenol,
(ii) a combination of about 0.01% to 0.20% (w/w) rosemary extract and 0.01% to 0.5% (w/w) synthetic or natural vitamin E and
(ii) a combination of about 0.01% to 1.0% (w/w) lecithin and of about 0.01% to 0.5% (w/w) synthetic or natural vitamin E, wherein % w/w refers to % w/w of the total composition

Even more preferred, the method of stabilizing THC in a composition comprising THC comprises
(a) preparing a solution of one or more members of the group consisting of rosemary extract, a combination of carnosic acid and carnosol, clove extract, eugenol, vitamin E (natural or synthetic), a mixture of alpha, beta, gamma- and delta tocopherol and lecithin in a lipophilic carrier;
   preferably of a combination of about 0.01% to 0.20% (w/w) rosemary extract and about 0.01% to 0.70% (w/w) eugenol,
   a combination of about 0.01% to 0.20% (w/w) rosemary extract and 0.01% to 0.5% (w/w) synthetic or natural vitamin E or
   a combination of about 0.01% to 1.0% (w/w) lecithin and of about 0.01% to 0.5% (w/w) synthetic or natural vitamin E,
   wherein % w/w refers to % w/w of the total composition and
(b) purging the solution obtained in (a) with N₂;
(c) preparing a solution of THC with the solution obtained in (b) under heating to about 70 °C±5°C and
(d) purging the solution obtained in (c) with N₂,
wherein the lipophilic carrier is preferably MCT oil.

Moreover, the present invention provides the use of one or more additives(s) as defined in (c) according to appended claim 1 for the stabilization of THC.

The disclosure made above in the context of the composition (e.g. preferred concentrations, combination of additives etc.) equally apply to the use.

### General Method for preparation of the compositions according to the present invention:

The stable THC compositions according to the present invention may be prepared in accordance with the following general method comprising the followings steps:
(1) Preparing a solution of the additive(s) in MCT oil under stirring at RT until the additives(s) are/ is completely dissolved in the MCT oil.
(2) Purging the additive solution obtained in step (1) with N₂.
(3) Homogenizing the THC concentrate at maximally 60°C±5°C until THC is completely dissolved in small amount of MCT oil/additive(s) obtained in step (2).
(4) Allowing the solution obtained in (3) to cool down slowly to about 40 to 50°C, preferably below 45°C.
(5) Transferring the homogenized THC/MCT/additive(s) obtained in step (4) into the remaining MCT/additive solution obtained in step (2) and continuously stirring at RT until a fully homogenized clear solution of MCT/THC/additive(s) is obtained, optionally removing any resulting particles by filtration.
(6) Purging the resulting solution with N₂.

Steps (3) to (6) are carried out substantially in the absence of oxygen, by monitoring and maintaining the oxygen level below 0.01%, preferably at 0.00%.

### Examples:

### Exemplary Process for the Preparation of formulation II

Formulation (II) according to the present invention as described in Table 2 below in particular may be prepared by a process comprises the following steps:

### Process steps outside a nitrogen cabinet

(1) The required amount of MCT oil (100g) is weighed into in a transparent 2L glass bottle by using a precision balance.
(2) 20 mg of additive 1 (rosemary extract) is then weighed and transferred into the bottle containing the MCT oil.
(3) 20 mg of additive 2 (vitamin E) is then weighed and also transferred into it to the bottle containing the MCT oil. The bottle is closed tightly.
(4) The bottle containing the mixture (MCT oil + rosemary extract + vitamin E) obtained in (3), above is gently stirred at RT until the antioxidants are fully dissolved in the MCT oil.

The following steps are carried out in a nitrogen cabinet/under inert gas, the oxygen level should be maintained below 0.01%, preferably at 0.00%:
(5) The bottle containing the MCT mixture (MCT oil + rosemary extract+ vitamin E) obtained in (4) above is opened and the solution is purged with gentle stream of N₂ for 15-30 minutes.
(6) The dronabinol is heated to approximately 70±5°C until the dronabinol turns into flowable liquid. Fill more N₂ into the nitrogen cabinet, if needed to maintain an oxygen level of below 0.01%, preferably of 0.00%.
(7) The required amount of dronabinol is then transferred directly to an empty glass bottle kept on a tared precision balance, the glass bottle containing dronabinol is then transferred to a heating plate set at 90±5°C (the temperature of the dronabinol must be kept at about 70±5°C).
(8) The MCT oil mixture (MCT oil + rosemary extract + vitamin E) obtained above in step (5) is then slowly transferred into the bottle containing dronabinol until it reaches the 100 ml mark.
(9) The thus obtained dronabinol solution in MCT oil (containing rosemary extract + vitamin E) is then warmed at 60±5°C under continuous stirring until it becomes fully homogenous, resulting in a yellow, clear solution. If needed the oil may be filtered to remove the suspended particles (e.g. using a polypropylene filter with 10 to 50 µm pore size such as a Rotilabo round filter type 112 A).
(10) After a clear solution is obtained the solution obtained above is allowed to cool down for 15 to 20 minutes to about 40 to 50°C, preferably below 45°C.
(11) The solution obtained in step (10) is then purged with N₂ for 15 minutes and tightly closed.

Formulations I and III of the present invention can also be prepared in accordance with the principles of the above exemplary process described for formulation II of the invention.

Thus, formulation I may be prepared as follows:

### Process steps outside a nitrogen cabinet

(1) The required amount of MCT oil (100g) is weighed into in a transparent 2L glass bottle by using a precision balance.
(2) 20 mg of additive 1 (rosemary extract) is then weighed and transferred it to the bottle containing the MCT oil.
(3) 20 mg of additive 2 (eugenol) is then weighed and also transferred into it to the bottle containing the MCT oil. The bottle is closed tightly.
(4) The bottle containing the mixture (MCT oil + rosemary extract + eugenol) obtained in (3), above is gently stirred at RT until the additives are fully dissolved in the MCT oil.

The following steps are carried out in a nitrogen cabinet/under inert gas, the oxygen level should be maintained below 0.01%, preferably at 0.00%:
(5) The bottle containing the MCT mixture (MCT oil + rosemary extract+ eugenol) obtained in (4) above is opened and the solution is purged with gentle stream of N₂ for 15-30 minutes.
(6) The dronabinol is heated to approximately 70°C±5°C until the dronabinol turns into flowable liquid. Fill more N₂ into the nitrogen cabinet, if needed to maintain an oxygen level of 0.00%.
(7) The required amount of dronabinol is then transferred directly to an empty glass bottle kept on a tared precision balance, the glass bottle containing dronabinol is then transferred to a heating plate set at 90±5°C (the temperature of the dronabinol must be kept at about 70±5°C).
(8) The MCT oil mixture (MCT oil + rosemary extract + eugenol) obtained above in step (5) is then slowly transferred into the bottle containing dronabinol until it reaches the 100 ml mark.
(9) The thus obtained dronabinol solution in MCT oil (containing rosemary extract+ eugenol) is then warmed at 60± 5°C under continuous stirring until it becomes fully homogenous, resulting in a yellow, clear solution. If needed the oil may be filtered to remove the suspended particles (e.g. using a polypropylene filter with 10 to 50 µm pore size such as a Rotilabo round filter type 112 A)
(10) After a clear solution is obtained the solution obtained above is allowed to cool down for 15 to 20 minutes to about 40 to 50°C, preferably below 45°C.
(11) The solution obtained in step (10) is then purged with N₂ for 15 minutes and closed tightly.

Formulation III of the invention may be prepared as follows:
Process steps outside a nitrogen cabinet
(1) The required amount of MCT oil (100g) is weighed into in a transparent 2L glass bottle by using a precision balance.
(2) 20 mg of additive 1 (lecithin) is then weighed and transferred it to the bottle containing the MCT oil.
(3) 20 mg of additive 2 (vitamin E) is then weighed and also transferred into it to the bottle containing the MCT oil. The bottle is closed tightly.
(4) The bottle containing the mixture (MCT oil + lecithin + vitamin E) obtained in (3), above is gently stirred at RT until the additives are fully dissolved in the MCT oil.

The following steps are carried out in a nitrogen cabinet/under inert gas, the oxygen level should be maintained at below 0.01%, preferably at 0.00%:
(5) The bottle containing the MCT mixture (MCT oil + lecithin+ vitamin E) obtained in (4) above is opened and the solution is purged with gentle stream of N₂ for 15-30 minutes.
(6) The dronabinol is heated to approximately 70±5°C until the dronabinol turns into flowable liquid. Fill more N₂ into the nitrogen cabinet, if needed to maintain an oxygen level of below 0.01%, preferably of 0.00%.
(7) The required amount of dronabinol is then transferred directly to an empty glass bottle kept on a tared precision balance, the glass bottle containing dronabinol is then transferred to a heating plate set at 90±5°C (the temperature of the dronabinol must be kept at about 70±5°C).
(8) The MCT oil mixture (MCT oil + lecithin + vitamin E) obtained above in step (5) is then slowly transferred into the bottle containing dronabinol until it reaches the 100 ml mark.
(9) The thus obtained dronabinol solution in MCT oil (containing lecithin+ vitamin E) is then warmed at 60± 5°C under continuous stirring until it becomes fully homogenous, resulting in a yellow, clear solution. If needed the oil may be filtered to remove the suspended particles (e.g. using a polypropylene filter with 10 to 50 µm pore size such as a Rotilabo round filter type 112 A)
(10) After a clear solution is obtained the solution obtained above is allowed to cool down for 15 to 20 minutes to about 40 to 50°C, preferably below 45°C.
(11) The solution obtained in step (10) is then purged with N₂ for 15 minutes and closed tightly.

Compositions comprising higher amounts of additives (antioxidants) such as e.g. 0.05% rosemary extract and 0.05% vitamin E may be prepared correspondingly.

### Examples of the formulations according to the present invention

The formulations of the present invention can be prepared in accordance with the method of preparation described above.

Table 1: Exemplary Formulations of the compositions

The following exemplary formulations have been prepared. All formulations 1 to 10 are formulated with Dronabinol 2.5% w/w in MCT oil (e.g. MCT Oil Type V Ph. Eur. 11.0) ad 100% and contain the following antioxidants to stabilize the formulation as set out in table 1 below and no further components:

**Table 1**

| | **Antioxidant 1** | **Concentration (% w/w)** | **Antioxidant 2** | **Concentration (% w/w)** | **Antioxidant 3** | **Concentration (% w/w)** |
|---|---|---|---|---|---|---|
| **1** | Rosemary Extract (RE) | 0.02 | - | - | - | - |
| | (*e.g. StoppOx^{®} RE 60, Organic - Rosemary Extract, AOT)* | | | | | |
| **2** | Eugenol (E) | 0.02 | - | - | - | - |
| | (*e.g. Food grade obtained from Sigma-Aldrich)* | | | | | |
| **3** | RE | 0.02 | Mixture of tocopherols | 0.02 | - | - |
| | | | *(e.g. Tocopherol 70, AOT)* | | | |
| **4** | RE | 0.02 | Vitamin E | 0.02 | - | - |
| | | | *(e.g.-all-ac-α-tocopherol obtained from Sigma Aldrich)* | | | |
| **5** | RE | 0.02 | Eugenol | 0.02 | - | - |
| **6** | RE | 0.05 | Eugenol | 0.05 | | |
| **7** | Vitamin E | 0.02 | Mixture of tocopherols | 0.02 | RE | 0.01 |
| **8** | Lecithin *(e.g. L-α-Lecithin, Soybean R&D grade from Sigma Aldrich)* | 0.04 | - | - | - | - |
| **9** | Lecithin | 0.02 | Vitamin E | 0.02 | - | - |
| **10 NRF*** | Palmitoyl ascorbic acid | 0.05 | - | - | - | - |

| | | | | | | |
|---|---|---|---|---|---|---|
| **Formulation 10 serves as a comparative example formulation.* | | | | | | |

For formulations 4, 7 and 9 synthetic vitamin E was used.

Table 2: Specific preferred compositions according to the present invention

Table 2 below depicts formulations I, II and III according to the present invention which are preferred, formulations II, III and IV are particularly preferred, whereby formulations II and IV are even more preferred, formulation IV is most preferred.

**Table 2**

| **Formulations** | **Component** | **Quality** | **Function** | **Weight %** |
|---|---|---|---|---|
| **Formulation** I | MCT oil | Ph. Eur | Diluent | 97.46% |
| | *(e.g. MCT Oil Type V Ph. Eur. 11.0)* | | | |
| | Rosemary extract | Food grade | Antioxidant /Stabiliser | 0.02% |
| | (*e.g. StoppOx^{®} RE 60, Organic-Rosemary Extract, AOT)* | | | |
| | Eugenol | Ph. Eur | Antioxidant /Stabiliser | 0.02% |
| | Dronabinol | DAC | Active ingredient | 2.5% |
| **Formulation II** | MCT oil | Ph. Eur | Diluent | 97.46% |
| | *(e.g. MCT Oil Type V Ph. Eur.11.0.)* | | | |
| | Rosemary extract | Food grade | Antioxidant /Stabiliser | 0.02% |
| | *(e.g. StoppOx^{®} RE 60, Organic-Rosemary Extract, AOT)* | | | |
| | Vitamin E | Ph. Eur. | Antioxidant /Stabiliser | 0.02% |
| | *(e.g. DL-all-rac-α-tocopherol, Sigma Aldrich)* | | | |
| | Dronabinol | DAC | Active ingredient | 2.5% |
| **Formulation III** | MCT oil | Ph. Eur. | Diluent | 97.46% |
| | Lecithin | Ph. Eur. | Antioxidant /Stabiliser | 0.02% |
| | Vitamin E | Ph. Eur. | Antioxidant /Stabiliser | 0.02% |
| | *(e.g. DL-all-rac-α-tocopherol, Sigma Aldrich)* | | | |
| | Dronabinol | DAC | Active ingredient | 2.5% |
| **Formulation IV** | MCT oil | Ph. Eur. | Diluent | 97.40% |
| | *(e.g. MCT Oil Type V Ph. Eur. 11.0.)* | | | |
| | Rosemary Extract | Food Grade | Antioxidant /Stabiliser | 0.05% |
| | *(e.g. StoppOx^{®} RE 60, Organic-Rosemary Extract, AOT)* | | | |
| | Vitamin E | PH. Eur. | Antioxidant /Stabiliser | 0.05% |
| | *(e.g. DL-all-rac-α-tocopherol, Sigma Aldrich)* | | | |
| | Dronabinol | DAC | Active ingredient | 2.5%% |

As set out above, the use of synthetic vitamin E is preferred in accordance with the present invention.

### Stability testing

The compositions according to the present invention and comparative composition according to the prior art (NRF) as exemplified in Table 1 above were tested for their stability (as measured by THC purity) in closed containers, continuously open containers and under "in use" conditions.

For all stability tests amber glass bottles (20 ml) with white caps and inserts were used ("containers"). These bottles are the envisaged primary packaging used for the finished product.

### 1. Stability testing in closed containers

Closed containers of the compositions were kept at three different temperatures
- RT (20°C±5°C)
- 40°C ("accelerated conditions")

Samples were tested with HPLC according to the stability time points (1 month, 3 months and 6 months). After each time point, a new/fresh bottle containing dronabinol solution was opened and 5µl of sample was taken and dissolved in 995µl of HPLC grade MeOH to perform HPLC analysis. Samples were injected and analyzed for THC purity degradation products of dronabinol (CBN & 8 ΔTHC) using a GraceSmart RP 18 column and isocratic elution. All the peaks appearing on the chromatogram were integrated except MCT oil and antioxidants.

**Table 3: Stability data for THC at RT, closed containers**

| **Formulation No** | **% original THC based on peak area purity** | **% original THC based on peak area purity** | **% original THC based on peak area purity** | **% original THC based on peak area purity** |
|---|---|---|---|---|
| | **t= 0** | **t= 1 month** | **t= 3 months** | **t= 6 months** |
| **1** | 99.1 | 98.95 | 98.81 | 98.61 |
| **2** | 99.1 | 98.79 | 97.9 | 96.74 |
| **3** | 99.1 | 98.9 | 98.8 | 98.69 |
| **4** | 99.1 | 99.1 | 98.9 | 98.84 |
| **5** | 99.1 | 99.1 | 98.9 | 98.85 |
| **6** | 99.0 | 98.85 | 98.65 | n.d. |
| **7** | 99.1 | 99.1 | 98.9 | 98.78 |
| **8** | 99.1 | 99.1 | 98.95 | 99.1 |
| **9** | 99.1 | 99.1 | 98.82 | 99.1 |
| **10*** | 99.1 | 99.1 | 98.9 | 98.9 |

| | | | | |
|---|---|---|---|---|
| **Formulation 10 serves as a comparative example formulation.* *n.d.= not determined* | | | | |

As is evident from the above data compositions of all formulations exhibit excellent stability at RT.

**Table 4: Stability data for THC at 40°C (accelerated conditions), closed containers**

| **Formulation No.** | **% original THC based on peak area purity** | **% original THC based on peak area purity** | **% original THC based on peak area purity** | **% original THC based on peak area purity** |
|---|---|---|---|---|
| | **t= 0** | **t= 1 month** | **t= 2 months** | **t= 6 months** |
| **1** | 99.1 | 98.4 | 97.4 | 85.2 |
| **2** | 99.1 | 98.9 | 71.21 *(t=3 months)* | n.d. |
| **3** | 99.1 | 98.9 | 97.9 | 91.89 |
| **4** | 99.1 | 98.55 | 97.5 | 94.96 |
| **5** | 99.1 | 98.82 | 97.9 | 89.55 |
| **6** | 99.0 | 98.04 | 95.15 *(t=3 months)* | n.d. |
| **7** | 99.1 | 99.1 | 97.99 | 91.88 |
| **8** | 99.1 | 99.1 | 98.38 | 94.69 |
| **9** | 99.1 | 99.1 | 98.66 | 95.46 |
| **10*** | 99.1 | 99.1 | 98.05 | 94.64 |

| | | | | |
|---|---|---|---|---|
| **Formulation 10 serves as a comparative example formulation.* *n.d.= not determined* | | | | |

As is evident from the above data compositions of formulation 4 (comprising Rosemary extract at 0.02% and Vitamin E at 0.0.2%) and of formulation 9 (comprising lecithin at 0.02% and Vitamin E at 0.02%) exhibit excellent stability under accelerated conditions.

### 2. Stability testing in open containers at RT

Dronabinol solution (2ml) from each formulation was transferred to transparent HPLC vials. Vials were kept at room temperature (20°C ± 5°C). Samples were collected once every week and analyzed with HPLC as described above and data was collected every week over a duration of 6 months.

**Table 5: Stability data for THC at RT, continuously open containers**

| **Formulation No.** | **% original THC based on peak area purity** | **% original THC based on peak area purity** | **% original THC based on peak area purity** | **% original THC based on peak area purity** |
|---|---|---|---|---|
| | **t=0** | **t=1 month** | **t=3 months** | **t= 6 months** |
| **1** | 99.1 | 99.1 | 97.37 | 87.3 |
| **2** | n.d. | n.d. | n.d. | n.d. |
| **3** | 99.1 | 98.56 | 98.22 | 96.34 |
| **4** | 99.1 | 98.88 | 98.49 | 97.43 |
| **5** | 99.1 | 99.01 | 99.01 | 89.5 |
| **6** | n.d. | n.d. | n.d. | n.d |
| **7** | 99.1 | 98.71 | 98.97 | 97.05 |
| **8** | n.d. | n.d. | n.d. | n.d. |
| **9** | 99.1 | 97.63 | 95.56 | 92.22 |
| **10*** | 99.1 | 98.9 | 98.2 | 97.6 |

| | | | | |
|---|---|---|---|---|
| **Formulation 10 serves as a comparative example formulation.* *n.d. = not determined* | | | | |

As is evident from the above, in particular the composition of formulation 4 (comprising Rosemary extract at 0.02% and Vitamin E at 0.02%) exhibits excellent stability when kept continuously in open containers at RT.

### 3. Stability testing under "in-use" conditions at RT

Compositions were kept at RT (20°C ± 5°C) and designated bottles as defined above for each composition/formulation were opened daily for retrieving 100µl samples of the corresponding compositions. 5 µl of each sample was dissolved in 995 µl of HPLC grade MeOH to perform HPLC analysis. Samples were injected and analyzed as described above. All peaks of the chromatogram were integrated except MCT oil and antioxidants.

**Table 6: Stability data for THC at RT, open, in-use containers**

| **Formulation No.** | **% original THC based on peak area purity** | **% original THC based on peak area purity** | **% original THC based on peak area purity** | **% original THC based on peak area purity** |
|---|---|---|---|---|
| | **t= 0** | **t= 1 month** | **t=3 months** | **t= 6 months** |
| **1** | 99.1 | 99.1 | 97.37 | 87.01 |
| **2** | 99.1 | 91.14 | n.d. | n.d. |
| **3** | 99.1 | 99.1 | 96.32 | 94.06 |
| **4** | 99.1 | 99.1 | 98.25 | 97.37 |
| **5** | 99.1 | 99.1 | 97.51 | 90.74 |
| **6** | 99.0 | 98.71 | 98.0 | n.d |
| **7** | 99.1 | 99.1 | 98.12 | 94.59 |
| **8** | 99.1 | 99.1 | 93.4 | 89.83 |
| **9** | 99.1 | 99.1 | 98.15 | 97.74 |
| **10*** | 99.1 | 99.1 | 98.5 | 97.35 |

| | | | | |
|---|---|---|---|---|
| **Formulation10 serves as a comparative example formulation.* *n.d.= not determined* | | | | |

As is evident from the above data compositions of formulation 4 (comprising Rosemary extract at 0.02% and Vitamin E at 0.02%) and of formulation 9 (comprising lecithin at 0.02% and Vitamin E at 0.02%) exhibit excellent stability under open, in-use conditions at RT.

Moreover, as is evident from the above tables the starting point for the stability tests was a purity of THC of 99.1% or 99.0%, respectively. The purity/content of THC must meet the DAC specification of THC > 95.0%.

It should be understood that the content of any documents cited herein, while not necessarily considered relevant for the patentability of this invention, is incorporated by reference in its entirety. More specifically, all referenced documents are incorporated by reference to the same extent as if each individual document was specifically and individually indicated to be incorporated by reference.

The invention being thus described, it will be obvious that the same may be varied in many ways. Such variations are not regarded as a departure from the spirit and scope of the invention, and all such modifications as would be obvious to one skilled in the art are intended to be included within the scope of the present invention/following claims.

## Claims

1. A composition comprising:
(a) delta-9-tetrahydrocannabinol (THC) in a concentration of from about 2.0% to about 50% (w/w) or from about 2.0 % (w/w) to about 25% (w/w) or from about 2.0% to about 20% (w/w);
(b) a lipophilic carrier in a concentration of from about 46.0% (w/w) to about 97.99% (w/w), wherein the lipophilic carrier is selected from grape seed oil, peanut oil, rape seed oil (canola oil), safflower oil, sunflower oil, sesame oil, coconut oil, palm oil, palm kernel oil, caprylic acid/capric acid triglycerides and medium chain triglyceride (MCT) oil, and
(c) at least one additive selected from the group consisting of (i) rosemary extract, (ii) a combination of carnosic acid and carnosol, (iii) clove extract, (iv) eugenol, (v) vitamin E, (vi) a mixture of α-, β-, γ-, δ- tocopherol and (vii) lecithin in a concentration of about 0.01% (w/w) to about 2.0 % (w/w);
wherein % (w/w) refers to % (w/w) of the total composition and wherein the THC and the additives are dissolved in the lipophilic carrier.

2. The composition according to claim 1, wherein the lipophilic carrier is medium chain triglyceride (MCT) oil.

3. The composition according to claims 1 or 2, wherein the composition comprises about 2.5 % (w/w) to about 5.0 % (w/w) THC.

4. The composition according to any of claims 1 to 3, wherein the composition comprises about 2.5% (w/w) THC.

5. The composition according to any of claims 1 to 4, wherein the composition comprises one or more of rosemary extract at a concentration of 0.01% to 0.20% (w/w), eugenol at a concentration of 0.01% to 0.70% (w/w), vitamin E at a concentration of 0.01% to 0.5% (w/w), a mixture of α-, β-, γ-, δ- tocopherol at a concentration of 0.01% to 0.5% (w/w) and/or lecithin at a concentration of 0.01% to 1.0% (w/w).

6. The composition according to any of claims 1 to 5, wherein the additive comprises:
(i) rosemary extract and eugenol,
(ii) rosemary extract and vitamin E,
(iii) rosemary extract and a mixture of α-, β-, γ-, δ- tocopherol,
(iv) vitamin E, a mixture of α-, β-, γ-, δ- tocopherol and rosemary extract,
(v) lecithin or
(vi) lecithin and vitamin E.

7. The composition according to any of claims 1 to 6, wherein the additive comprises a first and a second component selected from
(i) rosemary extract as the first component and eugenol as the second component,
(ii) rosemary extract as the first component and vitamin E as the second component,
(iii) rosemary extract as the first component and a mixture of α-, β-, γ-, δ- tocopherol as the second component, or
(iv) lecithin as the first component and vitamin E as the second component.

8. The composition according to any of claims 1 to 7, wherein the additive is selected from:
(i) a combination of about 0.01% to 0.20% (w/w) rosemary extract and about 0.01% to 0.70% (w/w) eugenol,
(ii) a combination of about 0.01% to 0.20% (w/w) rosemary extract and 0.01% to 0.5% (w/w) vitamin E and
(iii) a combination of about 0.01% to 1.0% (w/w) lecithin and of about 0.01% to 0.5% (w/w) vitamin E.

9. The composition according to any one of the preceding claims, selected from a composition comprising
(a) about 2.5% (w/w) THC,
(b) MCT oil and
(c) a combination of rosemary extract and eugenol;
a composition comprising
(a) about 2.5 % (w/w) THC,
(b) MCT oil and
(c) a combination of rosemary extract and vitamin E; and
a composition comprising
(a) about 2.5% (w/w) THC,
(b) MCT oil and
(c) a combination of lecithin and vitamin E.

10. The composition according to any of the preceding claims, wherein vitamin E is α-tocopherol.

11. The composition according to any of the preceding claims, wherein vitamin E is synthetic vitamin E (DL-all-rac-α-tocopherol).

12. A composition according to any of the preceding claims for the use in medicine.

13. The non-medicinal use of a composition according to any of claims 1 to 11.

14. A method of stabilizing THC in a composition comprising THC comprising:
(a) preparing a solution of one or more members of the group consisting of rosemary extract, a combination of carnosic acid and carnosol, clove extract, eugenol, vitamin E, a mixture of alpha, beta, gamma- and delta tocopherol and lecithin in a lipophilic carrier and
(b) purging the solution obtained in (a) with N₂;
(c) preparing a solution of THC with the solution obtained in (b) under heating to about 70°C±5°C and
(d) purging the solution obtained in (c) with N₂,
wherein the concentration of the of one or more members of the group consisting of rosemary extract, a combination of carnosic acid and carnosol, clove extract, eugenol, vitamin E, a mixture of alpha, beta, gamma- and delta tocopherol and lecithin in the total composition is as defined in (c) according to claim 1.

15. The use of one or more compound(s) as defined in (c) according to claim 1 for the stabilization of THC.
